# EUROPÄISCHE PATENTSCHRIFT

(11) **EP 1 165 055 B1**
(45) Veröffentlichungstag und Bekanntmachung des Hinweises auf die Patenterteilung: **19.10.2005**
(21) Anmeldenummer: 00934863.2
(22) Anmeldetag: 31.03.2000
(51) Int. Cl.: A61K 9/20, A61K 31/56, A61K 31/565

(54) **PHARMAZEUTISCHE ZUSAMMENSETZUNG ENTHALTEND ESTRADIOL ODER ETHNIYLESTRADIOL MIT EINEM EXTRUSIONSZUSATZSTOFF**
PHARMACEUTICAL COMPOSITION CONTAINING ESTRADIOL OR ETHINYLESTRADIOL AND AN EXTRUSION ADDITIVE
COMPOSITION PHARMACEUTIQUE COMPRENANT D'ESTRADIOL OU D'ETHINYLESTRADIOL AVEC UN ADDITIF D'EXTRUSION

(30) Priorität: 31.03.1999 DE 19916383
(43) Veröffentlichungstag der Anmeldung: 02.01.2002
(73) Patentinhaber: Schering Aktiengesellschaft, 13353 Berlin (DE)
(72) Erfinder: HÜLSMANN, Stefan, D-10405 Berlin (DE); BACKENSFELD, Thomas, D-13127 Berlin (DE)
(74) Vertreter: Schubert, Klemens
(86) Internationale Anmeldenummer: PCT/DE2000/001028
(87) Internationale Veröffentlichungsnummer: WO 2000/057853

(56) Entgegenhaltungen:
- WO-A-86/00802
- WO-A-93/11749
- WO-A-95/22319
- DE-A- 19 913 606
- DE-C- 19 705 538
- US-A- 5 618 560
- DATABASE WPI Section Ch, Week 199618 Derwent Publications Ltd., London, GB; Class A96, AN 1996-175670 XP002153273 & JP 08 053357 A (FUJI KAGAKU KOGYO KK), 27. Februar 1996 (1996-02-27)
- HULSMANN S. ET AL: "Melt extrusion. An alternative method for enhancing the dissolution rate of 17.beta.estradiol hemihydrate" EUR. J. OF PHARM. AND BIOPHARM., Bd. 49, Nr. 3, 2000, Seiten 237-242, XP000965152

## Beschreibung

Die vorliegende Erfindung betrifft pharmazeutische Zusammensetzungen, erhältlich durch Mischen mindestens eines Wirkstoffes mit mindestens einem Extrusionszusatzstoff aus der Gruppe der mit Fettsäuren veresterten Polyalkohole und gemeinsamer Schmelzextrusion.

Die Schmelzextrusion ist ein für die Herstellung pharmazeutischer Zusammensetzungen bekanntes Verfahren. Dabei werden im Stand der Technik für die verschiedenen Anwendungsgebiete sehr unterschiedliche Hilfsstoffe eingesetzt.

Das Schmelzextrusionsverfahren ist an sich universell einsetzbar, wobei einerseits Vormischungen, welche dann zu den gewünschten Arzneiformen weiterverarbeitet werden, hergestellt werden können oder direkt durch anschließende oder gleichzeitige Formgebung die gewünschten Arzneiformen erhältlich sind.

So beschreibt die US-A-5 618 560 eine pharmazeutische Zusammensetzung, welche durch Mischen von PEG mit PEG-400-monostearat und Gentamycinsulfat und anschließender Schmelzextrusion erhältlich ist.

JP 08 053357 A beschreibt orale pharmazeutische Zusammensetzungen, die man durch Mischen eines Argininsalzes mit einem Polyalkoholfettsäureester, wie Rohrzuckerfettester und anschließender Schmelzextrusion erhält.

Durch das Schmelzextrusionsverfahren hergestellte Tabletten enthaltend Vitamin C und Vitamin B12 sowie Glycerinmonostearat sind aus der DE 197 05 538 C bekannt.

In der WO 95/22319 A sind pharmazeutische Formulierungen hergestellt durch Schmelzextrusion beschrieben, die neben Clarithromycin, Glycerinbehenat sowie Polyvinylpyrrolidon enthalten.

In der WO 93/11749 A ist die Herstellung von Romglizon-Tabletten durch Schmelzextrusion beschrieben, die neben dem Wirkstoff Tween 80 und PEG enthalten.

WO 86/00802 A beschreibt die Herstellung von Clonidintabletten durch das Schmelzextrusionsverfahren, die außerdem PEG und PEG-400-monostearat enthalten.

In der WO-9625151 wird die Verwendung von niedrig substituierten wasserunlöslichen Hydroxypropylmethylcellulosen als Zusatzstoff bei der Schmelzextrusion beschrieben, wodurch die Wirkstofffreisetzung gezielt eingestellt werden kann.

So beschreibt die DE-OS-4418837 ein Schmelzextrusionsverfahren, welches lediglich für niedrigschmelzende pharmazeutische Wirkstoffe geeignet ist. Nach diesem Verfahren werden direkt tablettierbare Granulate erhalten, welche dann zu den gewünschten Arzneiformen weiterverarbeitet werden.

In der WO-9629061 wird ein Schmelzextrusionsverfahren für hochschmelzende, ionische pharmazeutische Wirkstoffe beschrieben, bei welchem die Wirkstoffe in ihrer nichtionischen Form zusammen mit einem Polymer und einem Salz schmelzextrudiert werden.

Ferner wird in der DE-OS-19531277 die Verwendung von Lipiden bei der Herstellung von festen Arzneiformen nach dem Schmelzextrusionsverfahren beschrieben. Hierbei verhindert der Zusatz der Lipide das Kleben der Mischung während der Schmelzextrusion und der sich anschließenden Formkalandrierung.

Die bekannten Verfahren und Zubereitungen weisen u. a. den Nachteil auf, daß nahezu für jeden Wirkstoff ein geeignetes oder optimales Extrusionsverfahren geschaffen werden muß. Dabei ist beispielsweise zu beachten, welchen Schmelzpunkt der Wirkstoff aufweist oder ob er in ionischer Form vorliegt.

Um eine ausreichende Freisetzung und "content uniformity" von niedrig dosierten und schlecht wasserlöslichen Wirkstoffen zu erzielen, kann eine Mikronisierung vorgenommen werden. Mikronisierte Arzneistoffe weisen jedoch erhebliche Nachteile auf. Neben der starken Staubbildung und der damit verbundenen Belastung für das Personal sind vor allem ein schwieriges Handling aufgrund von Luftadsorption und relativ niedrigen Dichten zu nennen. Außerdem zeigen mikronisierte Arzneistoffe auch oft die Tendenz zur Aggregation. Zudem ist die Mikronisierung mit hohen Kosten verbunden (Geräte, eigene Gebäude).

Ein weiteres Verfahren nach dem Stand der Technik beinhaltet die Verwendung von organischen Lösungsmitteln, wobei die Arzneistofflösung auf die verwendeten Hilfsstoffe aufgesprüht wird. Diese hat den Nachteil, daß die erforderliche Trocknung zu einer thermischen Belastung der Wirkstoffe führt. Auch sind nachteilige ökologische Folgen bei der Verwendung organischer Lösemittel gegeben.

Aufgabe der vorliegenden Erfindung ist es daher eine pharmazeutische Zusammensetzung zu schaffen, welche die Nachteile des Standes der Technik überwindet.

Gegenstand der vorliegenden Erfindung ist daher eine pharmazeutische Zusammensetzung, erhältlich durch Mischen mindestens eines Wirkstoffes mit mindestens einem Extrusionszusatzstoff aus der Gruppe der mit Fettsäuren veresterten Polyalkohole und gemeinsamer Schmelzextrusion, wobei der Extrusionszusatzstoff ein Zuckerfettsäureester, Polyethylenglycolfettsäureester oder ein Glycerolfettsäureester ist wobei mindestens einer der Wirkstoffe aus 17β-Estradiol oder Ethinyestradiol ausgewählt ist und als weiterer Hilfsstoff Polyvinylpyrrolidon, Polyethylenglycol oder Vinylpyrrolidon-Vinylacetat-Copolymer oder eine Mischung aus den genannten Stoffen enthalten ist. Dabei, sind die Polyalkohole Diole, Glykole, Glycerin, Mono-, Di- oder Oligosaccharide, Zuckeralkohole, Sorbit, Inosit, Pentaerythrit, Trimethylolpropan oder polymere Verbindungen mit mehreren Hydroxygruppen, Polyalkylenglykole, Polyethylenglykole, Polyether- und Polyesterpolyole sind und es ist sevorzugt daß die Fettsäuren 1 bis 31 Kohlenstoffatome aufweisen und unverzweigt und/oder verzweigt und/oder gesättigt und/oder ungesättigt sind.

Die Extrusionzusatztoffe weisen geringen Schmelzpunkt auf und/oder erniedrigen den Schmelzpunkt deren Mischung mit dem Wirkstoff derart, daß während der Extrusion die Mischung nicht zusätzlich erwärmt werden muß, sondern daß vielmehr der Preßdruck im Extruder zu der notwendigen Temperaturerhöhung ausreicht. Es ist daher bevorzugt, daß man die Schmelzextrusion ohne zusätzliche Zufuhr von Wärme durchführt.

Die Herstellung der Extrusionszusatzstoffe ist an sich bekannt. Sie sind durch Veresterung von Fettsäuren mit Polyalkoholen nach an sich bekannten Verfahren erhältlich.

Zur Veresterung geeignete Fettsäuren sind insbesondere gesättigte, unverzweigte Fettsäuren, wie Ameisen-, Essig-, Propion-, Butter-, Valerian-, Capron-, Önanth-, Capryl-, Pelargon-, Caprin-, Undecan-, Laurin-, Tridecan-, Myristin-, Pentadecan-, Palmitin-, Margarin-, Stearin-, Nonadecan-, Arachin-, Behen-, Lignocerin-, Cerotin-, Melissinsäure oder gesättigte, verzweigte Fettsäuren, wie Isobutter-, Isovalerian-, Tubercolostearinsäure oder ungesättigte, unverzweigte Fettsäuren, wie Acryl-, Croton-, Palmitolein-, Öl-, Eruca-, Sorbin-, Linol-, Linolen-, Elaeostearin-, Arachidon-, Clupanodon-, Docosahexaensäure.

Zur Veresterung geignete Polyole sind organische Verbindungen, die mindestens zwei alkoholische Hydroxy-Gruppen im Molekül enthalten, wie Diole, Glykole, Glycerin, Mono-, Di- oder Oligosaccharide, wie Glucose, Galactose, Mannose, Fructose, Arabinose, Xylose, Ribose, 2-Desoxyribose, Cellobiose, Maltose, Lactose, Saccharose, Gentiobiose, Melibiose, Trehalose, Turanose, Stachyose, Acarbose, Zuckeralkohole, wie beispielsweise Sorbit und Inosit, Pentaerythrit, Trimethylolpropan oder polymere Verbindungen, wie Polyalkylenglykole, Polyethylenglykole, Polyether- und Polyesterpolyole.

Geeignete Wirkstoffe sind beispielsweise:

Acebutolol, Acetylcystein, Acetylsalicylsäure, Aciclovir, Alprazolam, Albumin, Alfacalcidol, Allantoin, Allopurinol, Ambroxol, Amikacin, Amilorid, Aminoessigsäure, Amiodaron, Amitriptylin, Amlodipin, Amoxicillin, Ampicillin, Ascorbinsäure, Aspartam, Astemizol, Atenolol, Beclometason, Benserazid, Benzalkoniumhydroxid, Benzocain, Benzoesäure, Betametason, Bezafibrat, Biotin, Biperiden, Bisoprolol, Bromazepam, Bromhexin, Bromocriptin, Budesonid, Bufexamac, Buflomedil, Buspiron, Coffein, Campher, Captopril, Carbamazepin, Carbidopa, Carboplatin, Cefachlor, Cefalexin, Cefadroxil, Cefazolin, Cefixim, Cefotaxim, Ceftazidin, Ceftriaxon, Cefuroxim Axetil, Chloramphenicol, Chlorhexidine, Chlorpheniramine, Chiortalidon, Cholin, Ciclosporin, Cilastatin, Cimetidin, Ciprofloxacin, Cisaprid, Cisplatin, Clarithromycin, Clavulansäure, Clomibramin, Clonazepam, Clonidin, Clotrimazol, Clozapin, Codein, Colestyramin. Cromoglicinsäure, Cyanocobalamin, Cyproterone, Desogestrel, Dexamethason, Dexpanthenol, Dexthromethorphan, Dextropropoxiphen, Diazepam, Diclofenac, Digoxin, Dihydrocodein, Dihydroergotamin, Diltiazem, Diphenhydramin, Dipyridamol, Dipyrone, Disopyramid, Domperidon, Dopamin, Enalapril, Ephedrin, Epinephrin, Ergocalciferol, Ergotamin, Erythromycin, Estradiol, Ethinylestradiol, Etoposid, Eucalyptus Globulus, Famotidin, Felodipin, Fenofibrat, Fenoterol, Fentanyl, Flavinmononudeotid, Fluconazol, Flunarizin, Fluorouracil, Fluoxetin, Flurbiprofen, Furosemid, Gemfibrozil, Gentamicin, Ginkgo Biloba, Glibendamid, Glipizid, Glycyrrhiza Glabra, Guaifenesin, Haloperidol, Heparin, Hyaluronsäure, Hydrochlorothiazide, Hydrocodon, Hydrocortison, Hydromorphon, Ipratropiumhydroxid, Ibuprofen, Imipenem, Indomethacin, Iohexol, Iopamidol, Isosorbiddinitrat, Isosorbidmononitrat, Isotretinoin, Ketotifen, Ketoconazol, Ketoprofen, Ketorolac, Labetalol, Lactulose, Lecithin, Levocarnitin, Levodopa, Levoglutamid, Levonorgestrel, Levothyroxin, Lidocain, Lipase, Lisinopril, Loperamid, Lorazepam, Lovastatin, Medroxyprogesteron, Menthol, Methotrexat, Methyldopa, Methylprednisolon, Metoclopramid, Metoprolol, Miconazol, Midazolam, Minocyclin, Minoxidil, Misoprostol, Morphin, Multivitamine und Mineralien, Nystatin, N-Methylephedrin, Naftidrofuril, Naproxen, Neomycin, Nicardipin, Nicergoline, Nicotinamid, Nicotin, Nicotinsäure, Nifedipin, Nimodipin, Nitrendipin, Nizatidin, Norethisteron, Norfloxacin, Norgestrel, Nortriptylin, Ofloxacin, Omeprazol, Ondansetron, Pancreatin, Panthenol, Pantothensäure, Paracetamol, Penicillin G, Penicillin V, Phenobarbital, Pentoxifylline, Phenylephrin, Phenylpropanolamin, Phenytoin, Piroxicam, Polymyxin B, Povidone-Iod, Pravastatin, Prazosin, Prednisolon, Propafenon, Propranolol, Pseudoephedrin, Pyridoxin, Quinidin, Ramipril, Ranitidin, Reserpin, Retinol, Riboflavin, Rifampicin, Rutosid, Saccharin, Salbutamol, Salcatonin, Salicylsäure, Selegilin, Simvastatin, Somatropin, Sotalol, Spironolacton, Sucralfat, Sulbactam, Sulfamethoxazol, Sulpirid, Tamoxifen, Tegafur, Teprenon, Terazosin, Terbutalin. Terfenadin, Theophyllin, Thiamin, Ticlopidin, Timolol, Tranexamsäure, Tretinoin, Triamcinolonacetonid, Triamteren, Trimethoprim, Troxerutin, Uracil, Valproinsäure, Vancomycin, Verapamil, Vitamin E, Volinsäure, Zidovudin.

Die erfindungsgemäßen pharmazeutischen Zusammensetzungen sind feste Dispersionen, welche per se eine Reihe von Vorteilen aufweisen. Derartige feste Dispersionen sind zeigen beispielsweise ein verbessertes Auflösungsverhalten, was insbesondere bei in wäßrigen Medien schwer löslichen Wirkstoffen von Vorteil ist.

Die erfindungsgemäßen pharmazeutischen Zusammensetzungen weisen eine Reihe von Vorteilen auf. Die Zusammensetzung besitzt eine hohe Homogenität, insbesondere auch für niedrig dosierte Wirkstoffe, deren Gehalt beispielsweise weniger als 0,025 % bezogen auf die Einzeldosis beträgt. Dies ist insbesondere für niedrig dosierte Hormonpräparate von großer Bedeutung.

Die Wirkstoffe in den erfindungsgemäßen pharmazeutischen Zusammensetzungen weisen ferner eine hohe "content uniformity" auf, d. h. die Abweichung des Gehalts der Einzeldosis schwankt nur innerhalb geringer Grenzen. Auch dies ist insbesondere für niedrig dosierte Wirkstoffe ein großer Vorteil. Erfindungsgemäße pharmazeutische Zusammensetzungen erfüllen die von den Pharmakopöen (USP, Ph. Eur.) geforderten Grenzwerte.

Ein weiterer Vorteil der erfindungsgemäßen pharmazeutischen Zusammensetzungen ist es, daß die Lösungsgeschwindigkeit in Wasser schwer löslicher Wirkstoffe wesentlich verbessert ist und eine homogene Verteilung des Arzneistoffs erreicht wird, ohne die Wirkstoffe im Vorfeld zu behandeln.

Die beschriebene Erfindung stellt somit als eine Alternative z.B. zur Mikronisierung dar, wobei die Schmelzextrusion an sie gestellte Aufgaben - Verbesserung der Lösungsgeschwindigkeit und Erreichen einer "content uniformity" - wirtschaftlich in einem Schritt löst.

Ein weiterer Vorteil der erfindungsgemäßen pharmazeutischen Zusammensetzungen ist, daß die Lösungsgeschwindigkeit schwer löslicher Wirkstoffe wesentlich verbessert ist. Somit können auch derartige Wirkstoffe in eine Zubereitung eingebracht werden, welche den Wirkstoff rasch und nahezu vollständig innerhalb kurzer Zeit wieder freisetzt.

Es ist ferner von Vorteil, wenn die zu extrudierende Mischung bereits Hilfsstoffe enthält, die später als Tablettierhilfsstoffe nicht mehr zugesetzt werden müssen. Derartige extrudierte Massen können dann beispielsweise mit einer geringeren als der sonst üblichen Anzahl von Tablettierhilfsstoffen zu Tabletten weiterverarbeitet werden (z. B. Verzicht auf Schmiermittel).

Besonders vorteilhaft ist es ferner, daß auch thermolabile Wirkstoffe zu den erfindungsgemäßen pharmazeutischen Zusammensetzungen verarbeitbar sind. Da die Extrusionszusatzstoff niedrig schmelzen, kann bei geeigneter Wahl der Zusatzstoffe oft auf eine Erwärmung von außen verzichtet werden. In jedem Falle kann aber bei niedrigen Temperaturen, beispielsweise 40 bis 60 °C gearbeitet werden.

Ein weiterer Gegenstand der vorliegenden Erfindung ist ein Verfahren zur Herstellung pharmazeutischer Zusammensetzungen in dem man mindestens einen Wirkstoffes mit mindestens einem Extrusionszusatzstoff aus der Gruppe der mit Fettsäuren veresterten Polyalkohole mischt und die so erhaltene Mischung anschließend einer gemeinsamen Schmelzextrusion unterwirft.

Erfindungsgemäß bevorzugt ist es, daß man die Schmelzextrusion ohne Zufuhr von Wärme durchführt.

Der Menge der zugesetzten Extrusionszusatzstoffe ist an sich beliebig. Die für den jeweiligen Wirkstoff optimale Menge ist für den Fachmann mit wenigen Versuchen leicht zu ermitteln. Die Extrusionszusatzstoffe haben keine negativen Auswirkungen auf die Haltbarkeit der mit ihnen hergestellten Zusammensetzungen, Zubereitungen und Arzneimittel. Die Extrusionszusatzstoffe sind auch gut verträglich, da deren Abbau im Körper (Esterspaltung) zu nicht toxischen, sondern im allgemeinen zu physiologischen Stoffen führt.

Die erfindungsgemäß erhältlichen pharmazeutischen Zusammensetzungen können direkt als Arzneimittel dienen. Dabei kann es gegebenenfalls erforderlich sein, der Mischung aus der Wirkstoffe oder den Wirkstoffen mit dem oder den Extrusionszusatzstoffen weitere pharmazeutisch verträgliche Hilfs- und Zusatzstoffe zuzusetzen und diese Mischung dann der Schmelzextrusion zu unterwerfen. Dabei können beispielsweise auch Formkalander verwendet werden, um die gewünschten Arzneimttel zu erhalten.

Derartige Arzneimttel können erfindungsgemaß aber auch derart hergestellt werden, daß man weiterhin die extrudierte Mischung mahlt und mit weiteren pharmazeutisch verträglichen Hilfs- und Zusatzstoffen zu Arzneimitteln weiterverarbeitet.

Gegenstand der vorliegenden Erfindung sind somit auch Arzneimittel, enthaltend eine erfindungsgemäße pharmazeutische Zusammensetzung zusammen mit weiteren pharmazeutisch verträglichen Hilfs- und Zusatzstoffen.

Die folgenden Beispiele erläutern die Erfindung:

### Beispiel 1:

### Verbesserung der Lösungsgeschwindigkeit

Zusammensetzung eines Schmelzextrudates:

| | |
|---|---|
| 10% | 17-β-Estradiol (nicht mikronisiert) |
| 50% | PVP |
| 40% | Saccharose-monopalmitat |

Die Bestandteile werden gemischt. Diese Mischung wird einer Schmelzextrusion in einem Schneckenextruder mit einer Einlochdüse bei einer Extrusionstemperatur von 60°C und einer Schneckendrehzahl von 50Upm unterzogen.

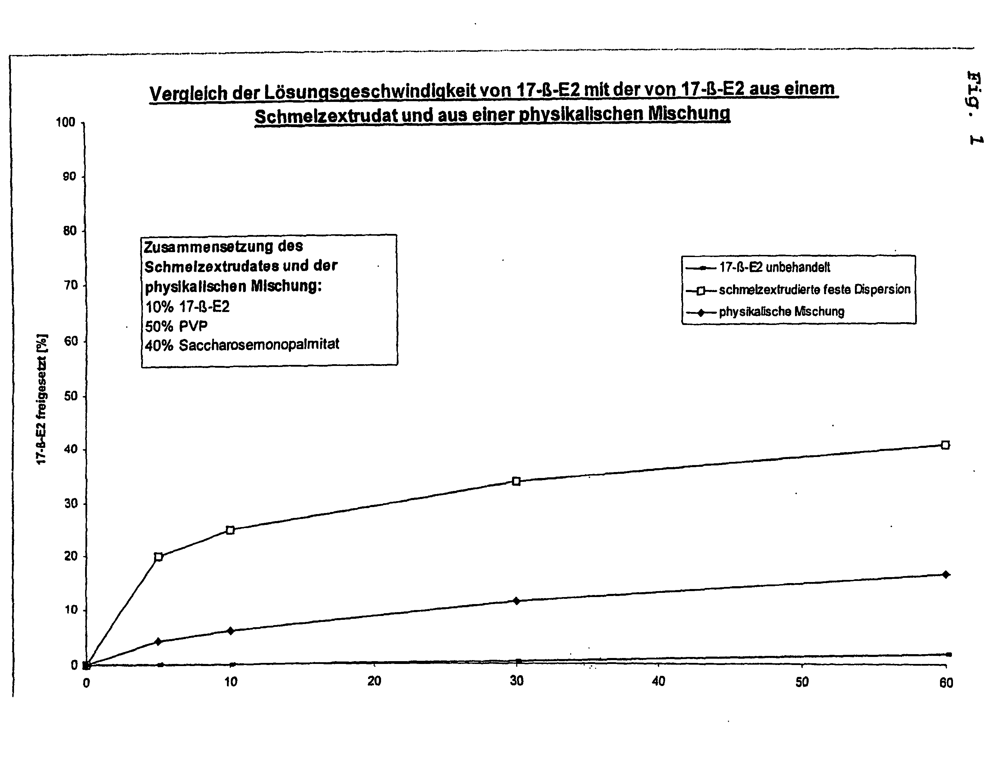

Die Lösungsgeschwindigkeit wird mittels Dissolutiontests (gemäß USP Apparatur II) in 900ml 0,1N Salzsäure bei 37°C über einen Zeitraum von 60min bestimmt (Drehzahl 100Upm) und mit der des reinen Wirkstoffs und einer physikalischen Mischung verglichen (Fig. 1).

Die Bestimmung des Gehalts an 17-β-Estradiol erfolgt per HPLC. Die Detektion erfolgt photometrisch bei einer Wellenlänge von 242 nm.

### Beispiel 2:

Zusammensetzung eines Schmelzextrudates:

| | |
|---|---|
| 10% | 17-β-Estradiol (nicht mikronisiert) |
| 50% | Vinylpyrrolidon-Vinylacetat-Copolymer |
| 40% | Mischung aus PEG-estern und Glycerolestern |

Extrusionstemperatur: 50°C; Schneckendrehzahl: 50 Upm

### Beispiel 3:

Zusammensetzung eines Schmelzextrudates:

| | |
|---|---|
| 30% | 17-β-Estradiol (nicht mikronisiert) |
| 30% | PEG 6000 |
| 40% | Saccharosemono- und distearat |

Extrusionstemperatur: 60°C; Schneckendrehzahl: 50 Upm

### Beispiel 4:

Verbesserung der Lösungsgeschwindigkeit einer applizierbaren Einzeldosis

Zusammensetzung eines Schmelzextrudates:

| | |
|---|---|
| 30% | 17-β-Estradiol (nicht mikronisiert) |
| 30% | PVP |
| 40% | Saccharose-monopalmitat |

Extrusionstemperatur: 60°C; Schneckendrehzahl: 50 Upm

Das erkaltete Schmelzextrudat wird zerkleinert und in einem Mischer mit Tablettierhilfsstoffen gemischt.

Zusammensetzung einer Pulverpreßmasse:

| | |
|---|---|
| 8,3 % | Schmelzextrudat mit 17-β-Estradiol |
| 45,6 % | mikrokristalline Cellulose |
| 45,6 % | Maisstärke |
| 0,5 % | Magnesiumstearat |

Es werden 80mg Tabletten mit einer Wirkstoffdosis von 2mg auf einer Tablettenpresse hergestellt.

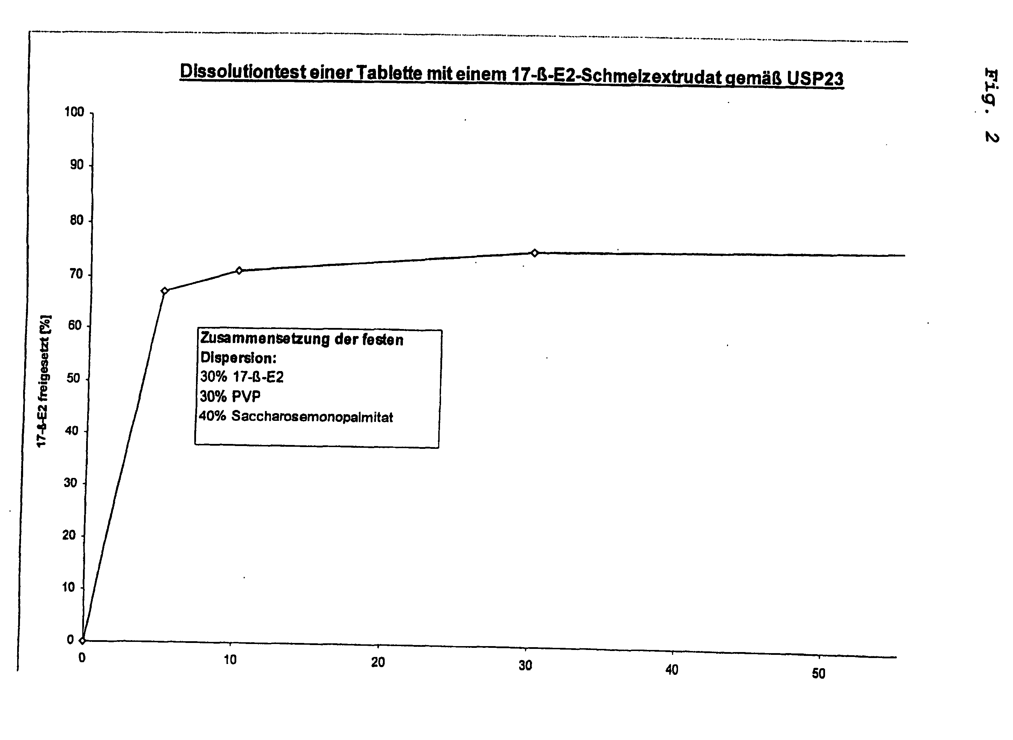

Die Freisetzungsgeschwindigkeit wird gemäß USP23-Monographie für Estradiol-Tabletten bestimmt (Fig. 2). Die Gehaltsbestimmung der Proben erfolgt per HPLC mit einer photometrischen Detektion bei 242 nm.

Die Anforderungen des Arzneibuchs an Estradiol-Tabletten hinsichtlich der Freisetzung werden erfüllt.

### Beispiel 5:

### Verwendung eines Schmiermittels als Extrusionszusatzstoff

Zusammensetzung eines Schmelzextrudates:

| | |
|---|---|
| 30% | 17-β-Estradiol (nicht mikronisiert) |
| 30% | PVP |
| 40% | Glycerol-tribehenat |

Extrusionstemperatur: 60°C; Schneckendrehzahl: 50 Upm

Bei einer anschließenden Tablettierung ist die Zugabe eines Schmiermittels nicht mehr notwendig.

### Beispiel 6:

### Erreichen einer homogenen Verteilung eines Arzneistoffes in einem Träger ("content uniformity")

Zusammensetzung eines Schmelzextrudates:

| | |
|---|---|
| 10% | Ethinylestradiol (nicht mikronisiert) |
| 50% | PVP |
| 40% | Saccharose-monopalmitat |

Extrusionstemperatur: 60°C; Schneckendrehzahl: 200 Upm

Die Untersuchung auf "content uniformity" wird gemäß USP23 durchgeführt. Es werden Tabletten bzw. feste Dispersionen mit einer Dosierung von jeweils 20µg untersucht. Die Prüfung auf "content uniformity" erfolgt mittels HPLC mit einer fluorimetrischen Detektion (Excλ =281nm; Emλ=305nm).

Die Ergebnisse entsprechen den Anforderungen der USP. Der Variationskoeffizient bei der Prüfung auf "content uniformity" liegt deutlich unter den geforderten 6%.

## Patentansprüche

1. Pharmazeutische Zusammensetzung, erhältlich durch Mischen mindestens eines Wirkstoffes mit mindestens einem Extrusionszusatzstoff aus der Gruppe der mit Fettsäuren veresterten Polyalkohole und gemeinsamer Schmelzextrusion, **dadurch gekennzeichnet, daß** der Extrusionszusatzstoff ein Zuckerfettsäureester, Polyethylenglycolfettsäureester oder ein Glycerolfettsäureester ist, wobei mindestens einer der wirkstoffe aus 17-β-Estradiol oder Ethinylestradiol ausgewählt ist und daß als weiterer Hilfsstoff Polyvinylpyrrolidon, Polyethylenglycol oder Vinylpyrrolidon-Vinylacetat-Copolymer oder eine Mischung aus den genannten Stoffen enthalten ist.

2. Pharmazeutische Zusammensetzungen gemäß Anspruch 1, **dadurch gekennzeichnet, daß** die Fettsäuren 1 bis 31 Kohlenstoffatome aufweisen und unverzweigt und/oder verzweigt und/oder gesättigt und/oder ungesättigt sind.

3. Pharmazeutische Zusammensetzung nach mindestens einem der voranstehenden Ansprüche, **dadurch gekennzeichnet, daß** man die Schmelzextrusion ohne zusätzliche Zufuhr von Wärme durchführt.

4. Pharmazeutische Zusammensetzung nach mindestens einem der Ansprüche 1 bis 3, erhältlich durch Mischen von 17-β-Estradiol, Polyvinylpyrrolidon und Saccharose-monopalmitat und gemeinsamer Schmelzextrusion bei 60 °C.

5. Pharmazeutische Zusammensetzung nach mindestens einem der Ansprüche 1 bis 3, erhältlich durch Mischen von 17-β-Estradiol, Polyvinylpyrrolidon und Glycerol-tribehenat und gemeinsamer Schmelzextrusion bei 60 °C.

6. Pharmazeutische Zusammensetzung nach mindestens einem der Ansprüche 1 bis 3, erhältlich durch Mischen von Ethinylestradiol, Polyvinylpyrrolidon und Saccharose-monopalmitat und gemeinsamer Schmelzextrusion bei 60 °C.

7. Verfahren zur Herstellung pharmazeutischer Zusammensetzungen in dem man mindestens einen Wirkstoffes mit mindestens einem Extrusionszusatzstoff aus der Gruppe der mit Fettsäuren veresterten Polyalkohole mischt und die so erhaltene Mischung anschließend einer gemeinsamen Schmelzextrusion unterwirft.

8. Verfahren gemäß Anspruch 7, **dadurch gekennzeichnet, daß** man die Schmelzextrusion ohne Zufuhr von Wärme durchführt.

9. Verfahren gemäß Anspruch 7 oder 8, **dadurch gekennzeichnet, daß** man weiterhin die extrudierte Mischung mahlt und mit weiteren pharmazeutisch verträglichen Hilfs- und Zusatzstoffen zu Arzneimitteln weiterverarbeitet.

10. Arzneimittel, enthaltend eine pharmazeutische Zusammensetzung nach Anspruch 1 zusammen mit weiteren pharmazeutisch verträglichen Hilfs- und Zusatzstoffen.

## Claims

1. Pharmaceutical composition obtainable by mixing at least one active ingredient with at least one extrusion additive from the group of polyalcohols esterified with fatty acids and joint melt extrusion, **characterized in that** the extrusion additive is a sugar fatty acid ester, polyethylene glycol fatty acid ester or a glycerol fatty acid ester, wherein at least one of the active ingredients is selected from 17-β-estradiol or ethinylestradiol and wherein as an additional adjuvant, polyvinylpyrrolidone, polyethylene glycol or vinylpyrrolidonevinyl acetate copolymer or a mixture that consists of the abovementioned substances is contained.

2. Pharmaceutical composition according to claim 1, wherein the fatty acids have 1 to 31 carbon atoms and are unbranched and/or branched and/or saturated and/or unsaturated.

3. Pharmaceutical composition according to claim 1 wherein the melt extrusion is carried out without additional heat input.

4. Pharmaceutical composition according to claim 1 obtainable by mixing 17-β-estradiol, polyvinylpyrrolidone and saccharose monopalmitate and joint melt extrusion at 60°C.

5. Pharmaceutical composition according to claim 1 obtainable by mixing 17-β-estradiol, polyvinylpyrrolidone and glycerol tribehenate and joint melt extrusion at 60°C.

6. Pharmaceutical composition according to claim 1 obtainable by mixing ethinylestradiol, polyvinylpyrrolidone and saccharose monopalmitate and joint melt extrusion at 60°C.

7. Process for the production of pharmaceutical compositions in which at least one active ingredient is mixed with at least one extrusion additive from the group of polyalcohols esterified with fatty acids, and the mixture that is thus obtained is then subjected to a joint melt extrusion.

8. Process according to claim 7, wherein the melt extrusion is carried out without heat input.

9. Process according co claim 7 or 8, wherein in addition, the extruded mixture is ground and further processed into pharmaceutical agents with additional pharmaceutically compatible adjuvants and additives.

10. Pharmaceutical agents that contain a pharmaceutical composition according to claim 1 together with additional pharmaceutically compatible adjuvants and additives.

## Revendications

1. Préparation pharmaceutique, pouvant être obtenue par mélange d'au moins une substance active avec au moins un additif d'extrusion du groupe des polyalcools estérifiés avec des acides gras et par extrusion en masse fondue commune, **caractérisée en ce que** l'additif d'extrusion est un ester d'acide gras de sucre, un ester d'acide gras et de polyéthylèneglycol ou un ester d'acide gras et de glycérol, au moins une des substances actives étant choisie parmi le 17-β-estradiol ou l'éthynylestradiol et **en ce que** de la polyvinylpyrrolidone, du polyéthylèneglycol ou un copolymère de vinylpyrrolidone-acétate de vinyle ou un mélange des substances mentionnées est contenu comme autre adjuvant.

2. Préparations pharmaceutiques selon la revendication 1, **caractérisées en ce que** les acides gras présentent 1 à 31 atomes de carbone et sont non ramifiés et/ou ramifiés et/ou saturés et/ou insaturés.

3. Préparation pharmaceutique selon au moins l'une quelconque des revendications précédentes, **caractérisée en ce qu'**on réalise l'extrusion en masse fondue sans apport supplémentaire de chaleur.

4. Préparation pharmaceutique selon au moins l'une quelconque des revendications 1 à 3, pouvant être obtenue par mélange de 17-β-estradiol, de polyvinylpyrrolidone et de monopalmitate de saccharose et par extrusion en masse fondue commune à 60°C.

5. Préparation pharmaceutique selon au moins l'une quelconque des revendications 1 à 3, pouvant être obtenue par mélange de 17-β-estradiol, de polyvinylpyrrolidone et de tribéhénate de glycérol et par extrusion en masse fondue commune à 60°C.

6. Préparation pharmaceutique selon au moins l'une quelconque des revendications 1 à 3, pouvant être obtenue par mélange d'éthynylestradiol, de polyvinylpyrrolidone et de monopalmitate de saccharose et par extrusion en masse fondue commune à 60°C.

7. Procédé pour la préparation de préparations pharmaceutiques, dans lequel on mélange au moins une substance active avec au moins un additif d'extrusion du groupe des polyalcools estérifiés avec des acides gras et on soumet ensuite le mélange ainsi obtenu à une extrusion en masse fondue commune.

8. Procédé selon la revendication 7, **caractérisé en ce qu'**on réalise l'extrusion en masse fondue sans apport de chaleur.

9. Procédé selon la revendication 7 ou 8, **caractérisé en ce qu'**on broie en outre le mélange extrudé et on le transforme ultérieurement avec d'autres adjuvants et additifs pharmaceutiquement acceptables en médicaments.

10. Médicament, contenant une préparation pharmaceutique selon la revendication 1 avec d'autres adjuvants et additifs pharmaceutiquement acceptables.
